# EUROPEAN PATENT SPECIFICATION

(11) **EP 1 254 673 B1**
(45) Date of publication and mention of the grant of the patent: **09.11.2005**
(21) Application number: 01131051.3
(22) Date of filing: 31.12.2001
(51) Int. Cl.: A61L 31/08, A61L 31/14, A61L 31/16, A61L 33/00

(54) **Immuno-tolerant stent with surface microstructure**
Immunotoleranter Stent mit Oberflächen-Mikrostruktur
Stent immunotolérant ayant une surface microstructurée

(30) Priority: 02.05.2001 US 847626; 12.10.2001 EP 01124528
(43) Date of publication of application: 06.11.2002
(73) Proprietor: InFlow Dynamics, Inc., Springfield, Virginia 22151 (US)
(72) Inventor: Alt, Eckhard Dr., 85521 Ottobrunn (DE)
(74) Representative: Vossius & Partner

(56) References cited:
- EP-A- 0 806 212
- WO-A-00/10623
- DE-A- 19 916 086
- US-A- 5 735 896

## Description

The invention refers to a stent according to the preamble of claim 1.

Stents are widely used to support a lumen in the human body, especially the lumen of a coronary artery.

The problem of coronary restenosis has been one of the major challenges for modern interventional cardiology.

Despite major progress in stent design, stent technology, flexibility, delivery systems and other means to improve the technique of coronary stenting, coronary restenosis still is of concern. Roughly 25% of all patients which receive an intervention in their vascular bed receive an implant and suffer a restenosis, which means that more than 50% of the initial lumen gain are lost in the follow-up between 6 weeks and 6 months.

A wide range of solutions have been proposed including a modification of the stent surface by iridiumoxide or the coating of the stent surface with biodegradable or non-biodegradable coatings. It has been proposed to provide the stent surface with a coating of an immuno suppressing drug, namely "Tacrolimus", which prevents or at least reduces the immuno-response by a modification of the immuno-response of the body. These drugs are otherwise used to suppress the rejection of transplants in the human body. In addition, recent research has shown, that a rough stent surface may be advantageous to prevent restenosis. However, precise predictions and results could not be achieved as yet.

WO 00/10623 discloses a stent with a surface pattern comprising a plurality of depressions with an average diameter of about 30 - 65 micrometers to achieve an organised growth pattern of infiltrating cells in order to avoid immuno-reactions of the body leading to restenosis.

It is an object of the present invention to propose an alternative stent design, which provokes a minimum immuno-reaction by the body against the implanted foreign body, thus avoiding restenosis after implantation.

This object is solved by the features of claim 1.

Further embodiments and advantages of the invention are disclosed in the subclaims.

The solution is, to minimize the surface recognition of the stent as an implanted foreign body creating a stent surface, which is more immuno tolerant than a smooth or generally rough surface. It has shown, that the stent restenosis primarily is a foreign body recognition with concomitant inflammatory reaction against the implanted foreign body, very similar to the rejection of a transplanted organ with a foreign body surface to the body. This reaction primarily is brought forward by T-Lymphocytes. These Lymphocytes attack anything which is determined to be foreign and a release of Cytokines is affected following this recognition.

It could be shown, that the reaction to a transplanted foreign body is minimized, if a certain stent surface structure can be achieved which mimics the surface structure of the patient's own cells. By applying a surface structure of the stent having microstructures with lateral dimensions in the range of o.5 to 5 micrometers (µm) and especially 1 to 3 µm roughness, the body is blinded towards the foreign body and by surface characterization and immuno tolerance is induced.

This is absolutely in contrary to the current opinion that a stent surface should be smooth in order to be well tolerated by the human body. The experiment, that has been conducted at applicants research lab, has shown, that especially a structure with an inert material, such as iridiumoxide, niobiumoxide, titaniumnitrate or other very inert ceramic-like structures, suppress this immuno-reaction.

In addition, small traces of the immuno modulating drug "Tacrolimus" or "Sirolimus" enhance the action towards a suppressed immuno reaction.

The consequences of this blinding of the immuno response by especially two factors, surface modification and in addition the adding of small traces of an immuno suppressing drug without any systemic effect, promote the healing of the stent without suppressing the proliferation of smooth muscle cells.

A problem of all currently proposed concepts with drugs was the assumption that only the inhibition of the proliferation would be sufficient to suppress restenosis. In contrary thereto, the aim of this invention is to allow the proliferation of smooth muscle cells to the same degree as they normally would proliferate, but suppress the continuous overshooting proliferation that goes beyond the wound healing. This overshooting proliferation is responsible for the restenosis and is based on an inflammatory response toward the foreign body which is recognized by the T-cell system.

The combined approach of blinding the recognition of the implanted foreign body by grading a structure in a range of 1 to 5µm, especially 1 to 3µm, by additionally using material that has very inert surface properties, has no release of toxic substances, has a positive surface charge in the range 3x10⁻² to 5x10⁻² N/m (30 to 50 dynes/cm (g x cm x s⁻²/cm)), which reduces fibrinogen adhesion, that also diminishes the inflammatory reaction towards the release of metallic salts from otherwise stainless steel, which is especially avoided by an inert ceramic-like structure, all these factors contribute to the diminished immuno response.

The additional application of an immuno suppressive drug such as Sirolimus or Tacrolimus helps to overcome the very weak inflammatory response toward such an implanted foreign body, which is not only due to the surface recognition, but also to the mechanical trauma which is brought forward by the implantation of the stent.

The application of the immuno drug can be effected either by dipping the stent in a solution of the drug. For example, 40 mg Tacrolimus are dissolved in 6 ml of Chloroform or Ethylacetate and on the stent surface soaked by adhesion forces and by capillary forces the drug into the surface coating. The stent is taken out of the solution and the solvent evaporates following the high pressure of the dissolved chloroform into the ambient atmosphere rapidly. The consequence is, that dependent on the initial concentration small traces of the drug are bound to the surface and soaked into the network, the grooves and the capillaries of the rough surface structure of the stent.

Thereby, the surface modification of the stent serves two purposes:
1. It blinds the recognition of the stent surface as a foreign body and
2. it facilitates and helps to retain traces in a range of 5 - 50 µg of immuno drug on the surface structure of the stent.

In addition the application of the drug by means of a biodegradable or non-biodegradable carrier can be done as well. So drugs had been described previously and are consisting of either biodegradable such as polylactic acid or non-biodegrable or non-erodable polymers such as polyurethane, polyvinylacetate or other.

Further details and advantages of the present invention are disclosed in the following description in connection with the annexed drawings, in which.
Fig. 1 is a perspective partial view of a stent according to the invention;
Fig. 2 is a schematic view of a part D of the surface of the stent; and
Fig. 3 is a section according to line III - III in Fig. 2.

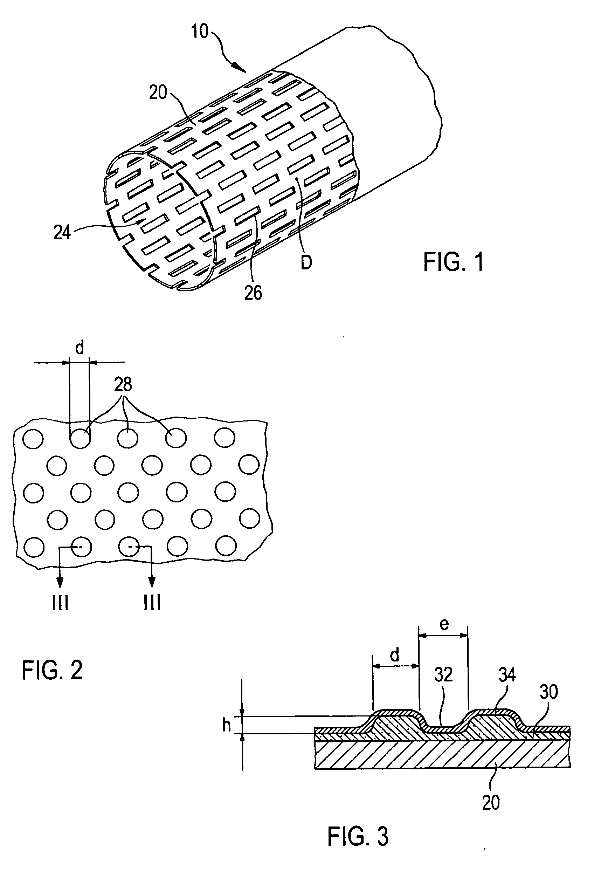

**Fig. 1** shows a part of a stent **10** in form of a small tube **20** preferably made of a niob-zirconium alloy which has two open ends (one is shown at **24**) and a lot of openings **26** in the wall of the tube **20,** so that the stent may be crimped on an angioplasty balloon (not shown) and deployed by the balloon on a treatment site for example in a coronary artery from its shown first small diameter to a second greater diameter so that the outer surface of the stent is in contact with the inner wall surface of the artery. Many designs of the stent are possible and well known in the state of the art; for the invention the configuration of the stent is not relevant.

The outer surface of the stent **10** is modified and provided with small protrusions or microstructures **28** as shown in **Figs. 2** and **3** for a small region **D** depicted in **Fig. 1.** These microstructures have a lateral extension **d** in the range of 0.5 to 5 micrometers, preferably in the range of 1 to 3 micrometers. The spacing **e** between adjacent microstructures is in the same range of 0.5 to 5 micrometers, preferably in the range of 1 to 3 micrometers, as schematically shown in **Fig. 3.** The height **h** of the microstructures between the peak of a microstructure and the valley adjacent to a next microstructure may have a lower value and lie in the range of 0.5 to 2 micrometers.

The microstructures **28** may be a coating **30** of a ceramic-like material such as iridiumoxide, which may be deposited on the surface of the surface of the stent by means of plasma or chemical vapour deposition or the like.

The microstructures **28** may have the form of microspheres or parts thereof; the surface of the microstructures may not be smooth and be provided with grooves or furrows **32** depending on the manufacturing process.

The stent is then dipped into a solution of an immuno suppressing drug, for example "Tacrolimus" or "Sirolimus", and a solvent such as chloroform, so that the solution covers the surface of the stent, partially by capillary forces in the region of the grooves **32.** The stent is then more or less covered by a coating **34** of the immuno suppressing drug.

## Claims

1. A stent for implantation in a lumen of the human body, the stent being expandable from a first small diameter to a second greater diameter, at which the stent is in contact with the inner surface of the lumen to hold the lumen open, whereby the stent is provided with a rough outer surface, **characterised in that** the surface of the stent is realised by microstructures separated from each other and having lateral extensions (d) in the range of 0.5 to 5 micrometers, whereby the recognition of the stent as an implanted foreign body is reduced.

2. Stent according to claim 1, **characterised in that** the lateral extensions (d) of the microstructures are in the range of 1 to 3 micrometers.

3. Stent according to claim 1 or 2, **characterised in that** the lateral distance between the microstructures from each other is in the range of 0.5 to 5 micrometers, especially 1 to 3 micrometers.

4. Stent according to one of the preceding claims, **characterised in that** the spacing (e) between adjacent microstructures is in the range of 0.5 to 5 micrometers, especially 1 to 3 micrometers.

5. Stent according to one of the preceding claims, **characterised in that** the surface structure of the stent is inert and releases no toxic substances.

6. Stent according to one of the preceding claims, **characterised in that** the surface structure of the stent has a surface charge in the range of 3x10⁻² to 5x10⁻² N/m (30 - 50 dynes/cm (g x cm x s⁻²/cm)).

7. Stent according to one of the preceding claims, **characterised in that** the stent is additionally provided with an immuno suppressing drug to treat the inner surface of the lumen and to prevent an immuno reaction thereof.

8. Stent according to claim 7, **characterised in that** the immuno suppressing drug is dissolved in a solvent and applied to the roughened surface of the stent by the capillary forces of the surface structure of the stent.

9. Stent according to claim 7, **characterised in that** the immuno suppressing drug adheres on the surface of the stent following the capillary forces without using a carrier.

10. Stent according to one of claims 7 to 9, **characterised in that** the addition of the immuno suppressing drug is realised by using the capillaries and grooves of the surface structure of the stent to retain the immuno suppressing drug on said surface.

11. Stent according to one of the preceding claims 7 to 10, **characterised in that** the drug is able to suppress an immuno reaction or response interacting with the T-cells of the human body.

12. Stent according to one of the preceding claims 7 to 11, **characterised in that** the immuno suppressing drug is sirolimus

13. Stent according to one of the preceding claims 7 to 11, **characterised in that** the immuno suppressing drug is tacrolimus.

14. Stent according to one of the preceding claims, **characterised in that** the surface of the stent modified by the microstructures is made of iridiumoxide.

15. Stent according to one of the preceding claims 1 to 13, **characterised in that** the surface of the stent modified by the microstructures is made of niobiumoxide.

16. Stent according to one of the preceding claims 1 to 13, **characterised in that** the surface of the stent modified by the microstructures is made of titanium nitrate.

## Patentansprüche

1. Stent zur Implantation in ein Lumen eines menschlichen Körpers, wobei der Stent von einem ersten kleinen Durchmesser zu einem zweiten größeren Durchmesser expandierbar ist, bei dem der Stent in Kontakt mit der Innenfläche des Lumens ist, um das Lumen offenzuhalten, wobei der Stent mit einer rauhen Außenfläche versehen ist, **dadurch gekennzeichnet, daß** die Oberfläche des Stents durch Mikrostrukturen realisiert ist, die voneinander beabstandet sind und seitliche Ausdehnungen (d) im Bereich von 0,5 bis 5 µm haben, wodurch die Erkennung des Stents als implantierter Fremdkörper reduziert ist.

2. Stent nach Anspruch 1, **dadurch gekennzeichnet, daß** die seitlichen Ausdehnungen (d) der Mikrostrukturen im Bereich von 1 bis 3 µm sind.

3. Stent nach Anspruch 1 oder 2, **dadurch gekennzeichnet, daß** der seitliche Abstand zwischen den Mikrostrukturen voneinander im Bereich von 0,5 bis 5 µm, insbesondere 1 bis 3 µm ist.

4. Stent nach einem der vorhergehenden Ansprüche, **dadurch gekennzeichnet, daß** der Abstand (e) zwischen benachbarten Mikrostrukturen im Bereich von 0,5 bis 5 µm, insbesondere von 1 bis 3 µm ist.

5. Stent nach einem der vorhergehenden Ansprüche, **dadurch gekennzeichnet, daß** die Oberflächenstruktur des Stents inert ist und keine toxischen Substanzen freisetzt.

6. Stent nach einem der vorhergehenden Ansprüche, **dadurch gekennzeichnet, daß** die Oberflächenstruktur des Stents eine Oberflächenladung im Bereich von 3x10⁻² bis 5x10⁻² N/m (30 bis 50 Dyn/cm (g x cm x s⁻²/cm)) hat.

7. Stent nach einem der vorhergehenden Ansprüche, **dadurch gekennzeichnet, daß** der Stent zusätzlich mit einem immunsuppressiven Medikament versehen ist, um die Innenfläche des Lumens zu behandeln und eine Immunreaktion derselben zu verhindern.

8. Stent nach Anspruch 7, **dadurch gekennzeichnet, daß** das immunsuppressive Medikament in einem Lösemittel gelöst ist und durch die Kapillarwirkung der Oberflächenstruktur des Stents auf die aufgerauhte Oberfläche des Stents aufgebracht wird.

9. Stent nach Anspruch 7, **dadurch gekennzeichnet, daß** das immunsuppressive Medikament an der Oberfläche des Stents als Folge der Kapillarwirkung ohne Verwendung eines Trägers haftet.

10. Stent nach einem der Ansprüche 7 bis 9, **dadurch gekennzeichnet, daß** der Zusatz des immunsuppressiven Medikaments unter Verwendung der Kapillaren und Rillen der Oberflächenstruktur des Stents realisiert wird, um das immunsuppressive Medikament auf der Oberfläche festzuhalten.

11. Stent nach einem der vorhergehenden Ansprüche 7 bis 10, **dadurch gekennzeichnet, daß** das Medikament in der Lage ist, eine Immunreaktion oder -antwort unter Zusammenwirkung mit den T-Zellen des menschlichen Körpers zu unterdrücken.

12. Stent nach einem der vorhergehenden Ansprüche 7 bis 11, **dadurch gekennzeichnet, daß** das immunsuppressive Medikament Sirolimus ist.

13. Stent nach einem der vorhergehenden Ansprüche 7 bis 11, **dadurch gekennzeichnet, daß** das immunsuppressive Medikament Tacrolimus ist.

14. Stent nach einem der vorhergehenden Ansprüche, **dadurch gekennzeichnet, daß** die durch die Mikrostrukturen modifizierte Oberfläche des Stents aus Iridiumoxid besteht.

15. Stent nach einem der vorhergehenden Ansprüche 1 bis 13, **dadurch gekennzeichnet, daß** die Oberfläche des Stents, die durch die Mikrostrukturen modifiziert ist, aus Niobiumoxid besteht.

16. Stent nach einem der vorhergehenden Ansprüche 1 bis 13, **dadurch gekennzeichnet, daß** die durch die Mikrostrukturen modifizierte Oberfläche des Stents aus Titannitrat besteht.

## Revendications

1. Stent destiné à être implanté dans une lumière du corps humain, le stent étant expansible d'un premier petit diamètre à un deuxième diamètre plus grand, au niveau duquel le stent entre en contact avec la surface interne de la lumière pour maintenir la lumière ouverte, moyennant quoi le stent est muni d'une surface externe rugueuse, **caractérisé en ce que** la surface du stent est réalisée par des microstructures séparées les unes des autres et ayant des extensions latérales (d) dans la plage de 0,5 à 5 micromètres, moyennant quoi la reconnaissance du stent en tant que corps étranger implanté est réduite.

2. Stent selon la revendication 1, **caractérisé en ce que** les extensions latérales (d) des microstructures sont dans la plage de 1 à 3 micromètres.

3. Stent selon la revendication 1 ou 2, **caractérisé en ce que** la distance latérale entre les microstructures est dans la plage de 0,5 à 5 micromètres, et notamment dans la plage de 1 à 3 micromètres.

4. Stent selon l'une quelconque des revendications précédentes, **caractérisé en ce que** l'espacement (e) entre les microstructures adjacentes est dans la plage de 0,5 à 5 micromètres, et notamment dans la plage de 1 à 3 micromètres.

5. Stent selon l'une quelconque des revendications précédentes, **caractérisé en ce que** la structure superficielle du stent est inerte et ne libère aucune substance toxique.

6. Stent selon l'une quelconque des revendications précédentes, **caractérisé en ce que** la structure superficielle du stent a une charge superficielle dans la plage de 3 x 10⁻² à 5 x 10⁻² N/m (30 à 50 dynes/cm (g x cm x s⁻²/cm)).

7. Stent selon l'une quelconque des revendications précédentes, **caractérisé en ce que** le stent est muni en outre d'un médicament immunosuppresseur pour traiter la surface interne de la lumière et pour empêcher une immunoréaction de celle-ci.

8. Stent selon la revendication 7, **caractérisé en ce que** le médicament immunosuppresseur est dissous dans un solvant et appliqué à la surface rendue rugueuse du stent par les forces capillaires de la structure superficielle du stent.

9. Stent selon la revendication 7, **caractérisé en ce que** le médicament immunosuppresseur adhère à la surface du stent suite aux forces capillaires sans utiliser de porteur.

10. Stent selon l'une quelconque des revendications 7 à 9, **caractérisé en ce que** l'ajout du médicament immunosuppresseur est réalisé en utilisant les capillaires et les rainures de la structure superficielle du stent pour retenir le médicament immunosuppresseur sur ladite surface.

11. Stent selon l'une quelconque des revendications précédentes 7 à 10, **caractérisé en ce que** le médicament est capable de supprimer une immunoréaction ou une réponse immunologique interagissant avec les lymphocytes T du corps humain.

12. Stent selon l'une quelconque des revendications précédentes 7 à 11, **caractérisé en ce que** le médicament immunosuppresseur est le sirolimus.

13. Stent selon l'une quelconque des revendications précédentes 7 à 11, **caractérisé en ce que** le médicament immunosuppresseur est le tacrolimus.

14. Stent selon l'une quelconque des revendications précédentes, **caractérisé en ce que** la surface du stent modifiée par les microstructures est composée d'oxyde d'iridium.

15. Stent selon l'une quelconque des revendications précédentes 1 à 13, **caractérisé en ce que** la surface du stent modifiée par les microstructures est composée d'anhydride niobique.

16. Stent selon l'une quelconque des revendications précédentes 1 à 13, **caractérisé en ce que** la surface du stent modifiée par les microstructures est composée de nitrate de titane.
